# EUROPEAN PATENT SPECIFICATION

(11) **EP 3 626 104 B1**
(45) Date of publication and mention of the grant of the patent: **06.07.2022**
(21) Application number: 19197714.9
(22) Date of filing: 17.09.2019
(51) Int. Cl.: A45C 15/06, A45D 44/00, A01K 13/00, A61L 2/10

(54) **STORAGE DEVICE**
SPEICHERVORRICHTUNG
DISPOSITIF DE STOCKAGE

(30) Priority: 19.09.2018 US 201862733366 P; 08.02.2019 KR 20190014971
(43) Date of publication of application: 25.03.2020
(60) Divisional application: 22172550.0
(73) Proprietor: LG Electronics Inc., Seoul 07336 (KR)
(72) Inventor: YOO, Hyunsun, Seoul 08592 (KR); KIM, Miju, Seoul 08592 (KR); KIM, Hyewon, Seoul 08592 (KR); PARK, Ilha, Seoul 08592 (KR); CHUNG, Wookjun, Seoul 08592 (KR); CHUN, Jaehung, Seoul 08592 (KR); EUN, Yousook, Seoul 08592 (KR); KIM, Joogyeom, Seoul 08592 (KR); KIM, Sungkyung, Seoul 08592 (KR); KIM, Myongsun, Seoul 08592 (KR)
(74) Representative: Vossius & Partner Patentanwälte Rechtsanwälte mbB

(56) References cited:
- CN-A- 107 343 697
- KR-A- 20130 116 994
- KR-A- 20170 119 434
- KR-B1- 101 816 704
- KR-U- 20090 007 699

## Description

### Field

The present invention relates to a storage device for storing a pet care device.

### Background

In recent years, the population of people raising pets has increased, in addition to attachment and interest in pets. As a result, pet care devices to manage and treat pets are being developed.

KR 2006-0007303 A and KR 2006-0116425 A (hereinafter "related art") disclose pet care devices. However, such pet care devices have various disadvantages, which the present disclosure solves.

KR 2006-0007303 A discloses a brush that emits infrared light on the skin of the pet while managing the hair of the pet. However, the brush may be easily contaminated because hair and bacteria of the pet remains in the brush after treating the pet. In addition, the brush has a structure such that pollutants in an outdoor space may easily attach to the brush and such that the brush may be easily damaged by an external impact. Therefore, there is a need for a storage device that can prevent pet brushes from being contaminated and protect the brushes from external impacts.

KR 2006-0116425 A discloses a hair brush used by a person and a hair brush storage device that sterilizes the hair brush stored therein with an ultraviolet light or washes the hair brush with a liquid. However, a fixing structure to fix the hair brush is weak so that the hair brush may be easily separated from the fixing structure by an external impact. In addition, foreign matter separated from the hair brush remains in the interior of the storage device and the hair brush is exposed to moisture, which may cause contamination. Therefore, there is a need for a storage device that can stably store a pet care device used for pets, which may have a hair size smaller than a hair size of a human and may be exposed to more bacteria than humans.

KR 101 816 704 B1 relates to a sterilization case for cosmetic brushes. The sterilization case comprises: an outer case; a PCB electronic means; a case lid; an inner case; and a lifting plate. Various cosmetic brushes, regardless of the thickness, can be stored and extracted conveniently, and various bacteria in the stored brushes can be sterilized by means of an LED ultraviolet lamp disposed inside the rear wall of the outer case.

An object of the present disclosure is to provide a storage device in which a pet care device that treats or manages a pet is stably stored.

An object of the present disclosure is to provide a storage device for storing a pet care device in a clean state.

An object of the present invention is to provide a storage device according to claim 1, that improves a convenience of the user using the pet care device.

The present invention provides a storage device configured such that a pet care device may be stored in the storage device. The storage device is configured to sterilize the pet care device or to clean foreign substances present in the storage device so that the pet care device may be stored in a clean state.

The storage device includes a sterilizer to sterilize a pet care device is stored therein. The storage device may include a cleaning module that can remove foreign matter scattered in an internal space where the pet care device is stored.

The storage device may be portable while the pet care device is stored therein, improving convenience.

Embodiments disclosed herein may provide a storage device to store a pet care device therein and prevent the pet care device from being damaged by an external impact or foreign matter in an external space. The pet care device may be stored in a clean state to reduce or prevent a spread of bacteria to the pet by the pet care device.

Embodiments disclosed herein may provide a storage device configured to be portable so that a user may quickly treat or manage a pet according to the pet's condition, which may improve the health of the pet and increase the life of the pet.

### BRIEF DESCRIPTION OF THE DRAWINGS

The embodiments will be described in detail with reference to the following drawings in which like reference numerals refer to like elements wherein:
FIG. 1 is a perspective view of a storage device according to a first embodiment;
Figures 2A and 2B are cross-sectional views showing a locking structure of the storage device;
FIG. 3 is a perspective view showing a state in which a plurality of devices are stored in the storage device ;
FIG. 4 is a plan view showing a state in which the storage device is opened;
FIG. 5 is an exploded view showing a configuration of a first case according to a first embodiment;
FIG. 6 is an exploded view showing the configuration of a second case according to the first embodiment;
FIGS. 7 and 8 are views showing a sterilization of a plurality of devices stored in the storage device ;
FIG. 9 is a view showing a control method of a sterilizer to sterilize an interior of the storage device according to the first embodiment;
Figures 10A and 10B are cross-sectional views showing a state of charging a care device for pets stored in the storage device;
FIG. 11 is a perspective view of a storage device according to a second embodiment; and
FIG. 12 is a view showing a state of cleaning an inside of the storage device according to the second embodiment.

### DETAILED DESCRIPTION OF THE INVENTION

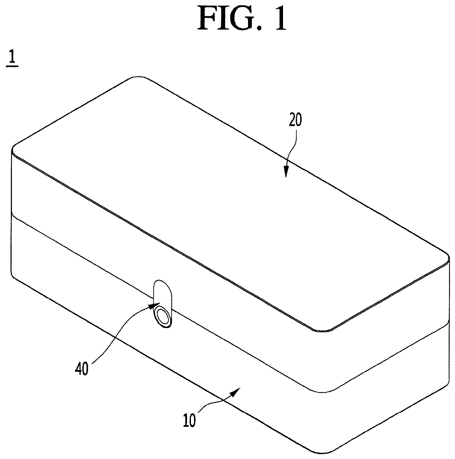

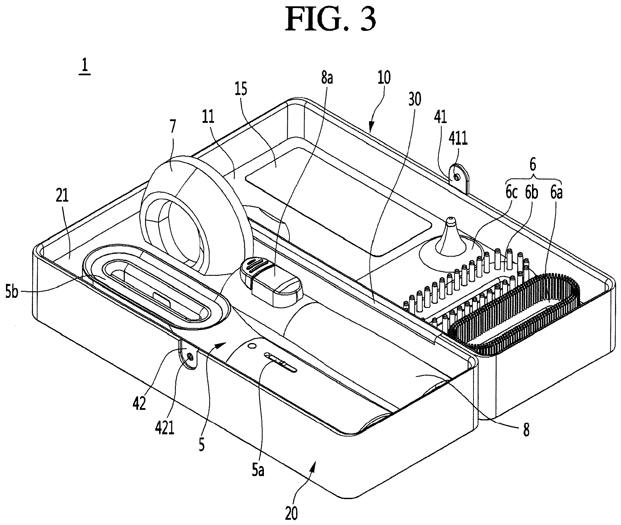

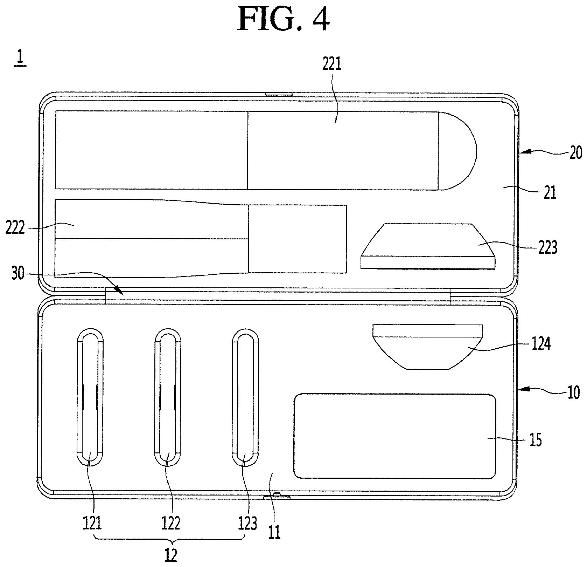

Referring to Figures 1 to 4, a storage device 1 has an internal space in which a device (e.g., a pet care device or tool) is stored. The pet care device may be a device to manage or treat a pet (e.g., hair brush, hair comb, skin sterilizer, or otoscope).

The storage device 1 includes a first or lower case 10 and a second or upper case 20. The first case 10 and the second case 20 are coupled to (e.g., hinged to) each other. When the first case 10 and the second case 20 are coupled to each other, the inner space is formed inside the first case 10 and the second case 20.

The storage device 1 may be formed as a three-dimensional polygon having a predetermined length, width, and height. The length may be longer than the width. The predetermined length, width, and height may be configured to accommodate a shape and dimensions of the pet care device stored in the internal space.

The storage device 1 includes a hinge 30 to connect the first case 10 and the second case 20 such that the first and second cases 10 and 20 are rotatable relative to each other. The hinge 30 may be provided at first sides (e.g., sides along a length direction) of the first case 10 and the second case 20. The first and/or second cases 10 and/or 20 may be rotated in a height direction (i.e., around an axis along the length direction) of the storage device 1 by the hinge 30. When the second case 20 rotates in an opening direction with respect to the first case 10 to open the storage device 1, the internal space of the storage device 1 may be exposed to an outside.

The storage device 1 may include a locking structure or device 40 to maintain a closed position of the first case 10 and the second case 20. The locking device 40 may be provided on second sides (e.g., along the length direction) opposite the first sides of the first case 10 and the second case 20. The locking device 40 may couple second sides of the first and second cases 10 and 20 so that the storage device 1 may remain closed.

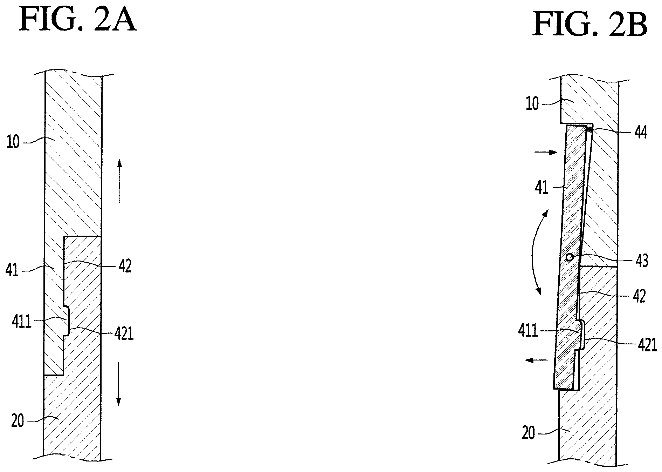

The locking device 40 may comprise an extension or rib 41 and an insertion groove 42. The rib 41 may be inserted into and coupled to the insertion groove 42 when the storage device 1 is closed. The rib 41 may be provided in one of the first case 10 and the second case 20 and the insertion groove 42 may be provided in the other of the first case 10 and the second case 20. The rib 41 may extend from one of the first case 10 and the second case 20 toward the other of the first case 10 and the second case 20. As exemplified in FIG. 2, the rib 41 may extend from the first case 10 to insert into the insertion groove 42, which may be formed in the second case 20.

The insertion groove 42 may be formed in a shape corresponding to a shape or outer contour of the rib 41. The insertion groove 42 may be recessed inward from an outer surface of the storage device 1. The rib 41 may be seated in the recessed insertion groove 42.

A locking protrusion or latch 411 may be formed on the rib 41. A protrusion groove 421 may be formed in the insertion groove 42 to have a shape corresponding to a shape or outer contour of the locking protrusion 411. The locking protrusion 411 may protrude inward from the rib 41. The protrusion groove 421 may be a portion of the insertion groove 42 that is recessed further inward than the rest of the insertion groove 42. When the rib 41 is seated in the insertion groove 42, the locking protrusion 411 may be inserted into the protrusion groove 421. When the locking protrusion 411 is inserted into the protrusion groove 421, the rib 41 may be prevented from being easily separated from the
insertion groove 42. The storage device 1 may be opened only when the locking protrusion 411 is separated from the protrusion groove 421 and the rib 41 is separated from the insertion groove 42.

The rib 41 may be coupled to the insertion groove 42 by an elastic force. As shown in FIG. 2A, a user may apply a force to overcome the elastic force and to separate the rib 41 from the insertion groove 42 in directions along the arrows.

As another example, the rib 41 may be rotatably provided in or attached to any one of the first case 10 and the second case 20. As shown in FIG. 2B, the rib 41 may include a rotation shaft 43 that forms a rotation center around which the rib 41 pivots or rotated toward and away from the first and second cases 10 and 20. A portion of the first or second case 10 or 20 in which the rib 41 is provided may be recessed inward from the outer surface. The locking protrusion 411 may be provided at a first end of the rib 41. An elastic member 44 may be provided at a second end of the rib 41 on a same surface as the locking protrusion 411 such that the elastic member 44 and the locking protrusion 311 face the first case 10 and the second case 20.

When the user presses the second end of the extension rib 41 having the elastic member 44, the first end of the rib 41 may rotate away from the first and second cases 10 and 20, and the locking protrusion 411 may be separated from the protrusion insertion groove 421. When the user not presses the second end of the extension rib 41, the locking protrusion 411 may rotate toward and be inserted into the protrusion groove 421 by a restoring force of the elastic member 44.A pet care device 5 may be stored inside the storage device 1. The pet care device 5 may be configured to manage pet hair or fur and/or treat pet hair or skin. The pet care device 5 may brush or comb hair, sterilize skin, and/or stimulate the skin for therapeutic purposes.

The pet care device 5 may include a body formed as a brush. The pet care device 5 may include a sterilizer 5b (e.g., ultraviolet or UV sterilizer) operated by an input device or operation switch 5a. The sterilizer 5b may include an ultraviolet light emitting device (e.g., light emitting diode or LED) to generate ultraviolet light (e.g., UVC light). The sterilizer 5b may irradiate ultraviolet rays to the skin of the pet to destroy harmful bacteria present in the skin. Since harmful bacteria may be eradicated by ultraviolet rays, the sterilizer 5b may prevent pet skin diseases caused by such harmful bacteria. The sterilizer 5b may be provided in a head or upper portion of a body of the pet care device 5.

The pet care device 5 may include a plurality of replacement modules or care tools 6 to be mounted. The care tools 6 may be selectively mounted and detached to the pet care device 5, and different care tools 6 may be used according to a condition of the pet. The plurality of care tools 6 may be mounted and detached on the head of the body of the pet care device 5. The plurality of care tools 6 may include a brush module 6a, a skin sterilization or comb module 6b, an ear sterilization module or otoscope 6c, etc. The care tools 6 may also be referred to as a first care tool 6a, second care tool 6b, and third care tool 6c. The brush module 6a may be a module to groom or brush pet hair. The skin sterilization module 6b may be a module to sterilize or stimulate the skin of a pet by irradiating ultraviolet rays to the skin of the pet. The ear sterilization module 6c may be a module to sterilize or treat an inside of a pet's ear.

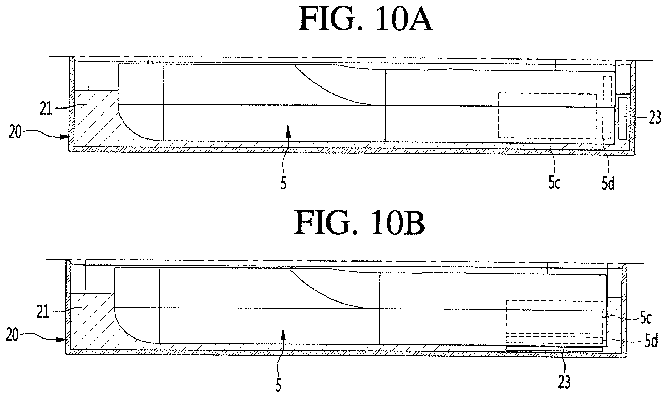

The pet care device 5 may include a base or support 7 to charge a care device battery 5c (FIG. 10) provided in the pet care device 5. The pet care device battery 5c may be charged while being supported by the base 7. The base 7 may charge the care device battery 5c via a wireless method (e.g., a wireless power transfer or WPT method) or a wired method (e.g., via a universal serial bus or USB charging cable). The base or support 7 may also be referred to as a cradle.

The pet care device 5 may include an extension or cleaning module 8 to suction or vacuum hair from the pet, floor, and/or pet care device 5. The cleaning module 8 may be a module to connect the pet care device 5 to a vacuum cleaner or robot cleaner. During grooming via the pet care device 5, pet hair or fur may be separated from the pet and collected in a dust bin of the vacuum cleaner via the cleaning module 8. The cleaning module 8 may be further provided with a replacement or connector switch 8a so that the cleaning module 8 may be easily pulled and separated from the pet care device 5 and the vacuum cleaner. The pet care device 5 may be further equipped with various modules in addition to the above, but for convenience of description, a storage device 1 based on the above-mentioned modules will be described.

The pet care device 5, the plurality of care tools 6, the base 7, and the cleaning module 8 may be housed in an interior space of the storage device 1. As an example, the pet care device 5, the cleaning module 8, and a portion of the base 7 may be fixed to or provided in the second case 20, while the plurality of care tools 6 and a remaining portion of the base 7 may be fixed to or provided in the first case 10. Fixed positions of the pet care device 5, the plurality of care tools 6, the base 7, and the cleaning module 8 may be variously changed.

The first case 10 includes a first internal fixing body or first body 11 in which the plurality of care tools 6 and a first portion of the base 7 may be provided. The first body 11 may also be referred to as a first inner body. The first body 11 may be inserted into and fixed to (e.g., adhered, fused, or screwed to) an inner side of the first case 10. When the first body 11 is fixed inside the first case 10, a plurality of electronic components or devices may be accommodated under the first body 11 between the first case 10 and the first body 11.

A height of the first body 11 may be lower than a height of the first case 10, while a length and a width of the first body 11 may correspond to the length and width of the first case 10. When the first body 11 is inserted into the first case 10, a height difference may be formed between the first body 11 and the first case 10. A portion of a bottom surface of the first body 11 may contact an inner bottom surface of the first case 10. A side surface of the first body 11 may contact an inner side surface of the first case 10.

A plurality of seating cavities or grooves 12 may be formed in the first body 11. The seating grooves 12 may also be referred to as seating spaces or recesses. The plurality of seating grooves 12 may include a first care tool seating groove 121, a second care tool seating groove 122, and a third care tool seating groove 123. The first to third care tool seating grooves 121 to 123 may be partially recessed from an upper or inner surface of the first body 11 so that a part of the first to third care tools 6a to 6c may be inserted therein, respectively. The first to third care tool seating grooves 121 to 123 may be configured to have shapes corresponding to shapes or outer contours of the first to third care tools 6a to 6c, respectively.

The first to third care tool seating grooves 121 to 123 may extend in a width direction of the storage device 1 and may be spaced apart from each other in the longitudinal direction of the storage device 1. The first to third care tool seating grooves 121 to 123 may be positioned to be concentrated or adjacent to each other on a first side or portion (e.g., left side) of the first body 11. A first base seating groove 124 and a light transmitting body or light diffuser 15 to be described later may be provided in a second side or portion (e.g., right side) of the first body 11. When the first to third care tool seating grooves 121 to 123 are concentrated on the first side of the first body 11, a space to accommodate the plurality of electronic components may be formed under the second side of the first body 11.

The plurality of care tool seating grooves 12 may include the first base seating groove 124. The first body 11 includes the light diffuser 15. The first base seating groove 124 and the light diffuser 15 may be adjacent to each other on the second side of the first body 11. The first base seating groove 124 may be formed by recessing the upper or inner surface of the first body 11. A first portion of the base 7 may be inserted into the first base seating groove 124 to be fixed in the first base seating groove 124.

The light diffuser 15 may define a surface or area through which ultraviolet rays for sterilizing the internal space of the storage device 1 are irradiated. The light diffuser 15 may be a light guide or light guide plate, and may be referred to as a light transmitter or light guide plate. The light diffuser 15 may transmit ultraviolet rays irradiated to the light diffuser 15 into the inner space of the storage device 1. The light diffuser 15 may be fixed within a mounting hole or opening 14 in which a part of the first body 11 may be opened. The mounting hole 14 may be formed in a shape corresponding to a shape of the light diffuser 15. The diffuser 15 may be made of a transparent or translucent material or a material having excellent ultraviolet transmittance.

The second case 20 includes a second body 21 in which the pet care device 5, the cleaning module 8, and a second portion of the base 7 may be provided. The second body 21 may be referred to as a second inner body or a second fixing body. The second body 21 may be inserted into and fixed (e.g., adhered, fused, screwed, or bolded) to an inner side of the second case 20. A height of the second body 21 may be less than a height of the second case 20, while a length and width of the second body 21 may correspond to the length and width of the second case 20. When the second body 21 is inserted into the second case 20, a height difference may be formed between the second body 21 and the second body 21. A portion of an upper or outer surface of the second body 21 may contact an inner upper surface of the second case 20. A side of the second body 21 may be in contact with an inner side of the second case 20.

The second body 21 may include a plurality of seating grooves or spaces 22. The plurality of seating grooves 22 may be referred to as seating recesses or cavities. The plurality of seating grooves 22 may include a care device seating groove 221, a cleaning module seating recess 222, and a second base seating groove 223. The care device seating groove 221, cleaning module seating groove 222, and second base seating groove 223 may each be recessed from a lower or inner surface of the second body 21 so that the pet care device 5, cleaning module 8, and second portion of the base 7 may be inserted therein, respectively. The inner surface of the second body 21 may face the inner surface of the first body 11 when the storage device 1 is closed. Shapes of the care device seating groove 221, cleaning module seating groove 222, and second base seating groove 223 may correspond to shapes or outer contours of the pet care device 5, cleaning module 8, and second portion of the base 7, respectively.

The second base seating groove 223 may be provided at a position corresponding to the first base seating groove 124 so that, when the seating device 1 is closed, the first base seating groove 124 vertically aligns with the second base seating groove 223, and the base 7 may provided in both the first and second base seating grooves 124 and 223. The care device seating groove 221 and the cleaning module seating groove 222 may extend in a length direction (i.e., the longitudinal direction) of the storage device 1.

When the storage device 1 is closed, for convenience of description, the upper and lower surface of the first and second bodies 11 and 21 will be described as if the second case 20 is closed on top of the first case 10. In such a configuration, the upper (or outer) surface of the second body 21 is attached to the upper inner surface of the second case 20, the lower (or outer) surface of the first body 11 is attached to the lower inner surface of the first case 10, and the upper (or inner) surface of the first body 11 faces the lower (or inner) surface of the second body 21. However, one of ordinary skill in the art will appreciate that the first case 10 may be closed on top of the second case 20 when the storage device 1 is closed.

When the storage device 1 is closed so that the upper surface of the first body 11 and the lower surface of the second body 21 face each other, ultraviolet rays passing through the light diffuser 15 may be provided to sterilize the pet care device 5, the plurality of care tools 6, the base 7, the cleaning module 8, and any additional devices stored in the storage device 1..

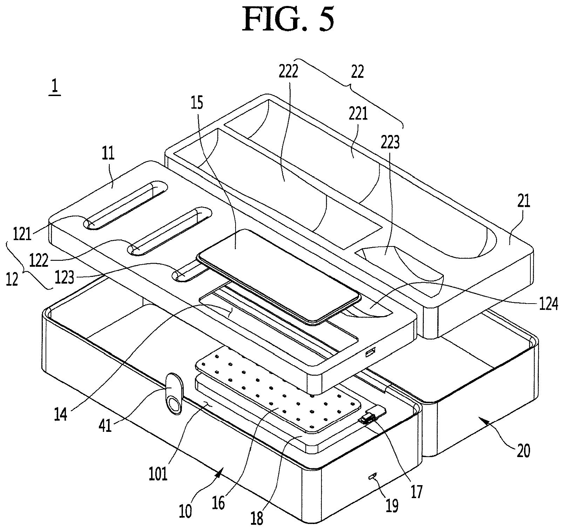

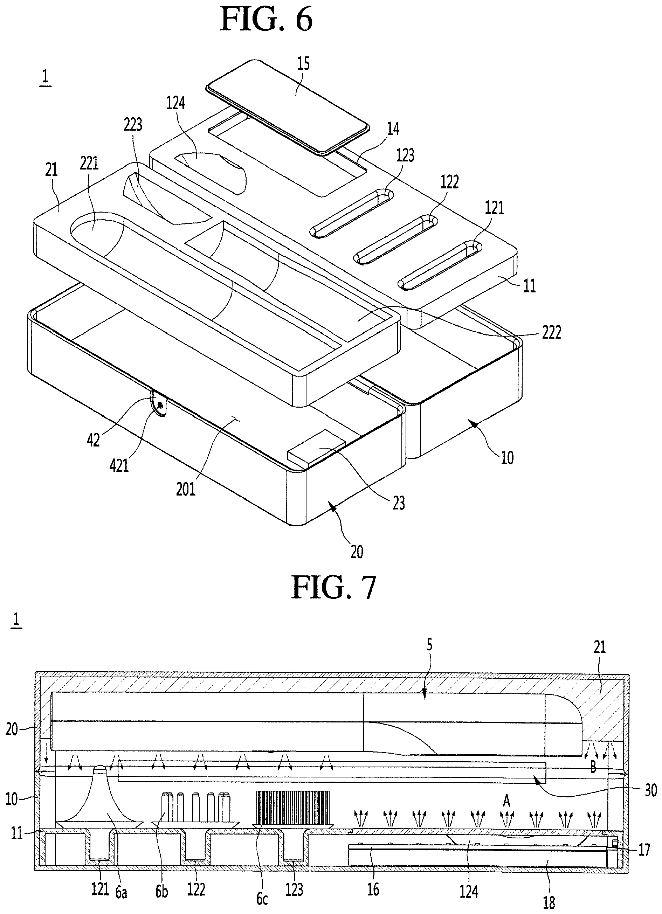

Referring to Figures 5 and 6, a first accommodating space 101 may be formed between the first case 10 and the first body 11 to accommodate a plurality of electronic devices or components. A second accommodation space 201 may be formed between the second case 20 and the second body 21. The light diffuser 15 may be provided to be adjacent to the first space 101, and the care device seating groove 221 may be provided on an inner side of the second accommodation space 201.

A sterilizer 16 is provided in the first accommodation space 101. The sterilizer 16 may be understood as an ultraviolet (UV) light (e.g., UV light emitting diode or LED) to generate ultraviolet light. The sterilizer 16 may include a plurality of light emitting devices to generate ultraviolet light and a printed circuit board (PCB) connected to the light emitting devices to form an electric circuit.

The sterilizer 16 may be provided at an outer or lower side (i.e., below) the light diffuser 15. Ultraviolet light emitted from the sterilizer 16 may be injected into a lower side of the light diffuser 15 and irradiated to an upper side of the light diffuser 15. The lower side of the light diffuser 15 may be a surface of the light diffuser 15 that faces the first accommodation space 101. The upper side of the light diffuser 15 may be a surface of the light diffuser 15 that faces a storage space between the first body 11 and the second body 21. The sterilizer 16 and the light diffuser 15 may be provided such that a large amount of ultraviolet light is introduced into the storage space between the first body 11 and the second body 21.

The first accommodation space 101 may include a circuit board device or printed circuit board 17. The circuit board 17 may charge a storage device battery 18 to be described later. In addition, the circuit board 17 may control an operation of the storage device 1 and an operation of the sterilizer 16. The circuit board 17 may be charged with external power supplied from an external or commercial power source (e.g., wall socket) to charge the storage device battery 18. The first case 10 may include a charging terminal 19 electrically connected or coupled to the circuit board 17. The charging terminal 19 may be exposed to an outside through a corresponding opening of the first case 10. A cable may be connected to the charging terminal 19, and the charging terminal 19 may receive the external power through the cable. Alternatively or in addition thereto, the charging terminal 19 may be exposed to the outside through a portion of the second case 20.

The storage device battery 18 may be provided in the first accommodation space 101 and charged via external power supplied through the cable and charging terminal 19. The storage device 1 may be operated by the power charged in the storage device battery 18. The storage device battery 18 may supply power to generate ultraviolet light from the sterilizer 16. The storage device battery 18 may also charge the pet care device 5 stored in the storage device 1.

Alternatively or in addition thereto, the first accommodation space 101 may further include a control circuit board or printed circuit board to control an operation of the storage device 1. Such a control circuit board may be electrically connected or coupled to the circuit board 17 and the storage device battery 18. "Electrically connected" or "electrically coupled" may mean connected by wire or wirelessly to transmit and receive power or signals (e.g., via a wireless power transfer or WPT method). The control circuit board may generate a control signal to control an operation of the storage device 1.

The second accommodation space 201 may include a power transmitter 23. The power transmitter 23 may be electrically connected or coupled to the storage battery 18 and the circuit board 17. The power transmitter 23 may transmit power to the pet care device 5 provided in the care device seating groove 221. The pet care device 5 may be provided with a power receiver 5d (FIG. 10) to receive power transmitted from the power transmitter 23. The power transmitter 23 may transmit power (e.g., via a wireless power transfer or WPT method) to the power receiver 5d when the pet care device 5 for pets is mounted in the care device seating groove 221. The care device seating groove 221 may be provided with a sensor to detect whether the pet care device 5 is mounted in the care device seating groove 221, and an operation of the power transmitter 23 may be controlled according to whether the pet care device 5 is mounted. In addition, the power transmitter 23 may be configured to transmit power continuously or, alternatively, periodically.

Positions of a plurality of electronic components accommodated in the first accommodation space 101 and the second accommodation space 201 may be changed. A plurality of electronic components provided in the first accommodation space 101 may be electrically connected or coupled to a plurality of electronic components provided in the second accommodation space 201, and the plurality of electronic components in the first and second accommodation spaces 101 and 201 may operate regardless of an arrangement or positions of the plurality of electronic components.

Referring to Figures 7 to 9, when the storage device 1 is opened, the pet care device 5, the plurality of care tools 6, and the cleaning module 8 may be stored in the storage space between the first and second bodies 11 and 21. When the storage device 1 is closed, the sterilizer 16 may operate to irradiate ultraviolet light to the storage space of the storage device 1.

Ultraviolet light A generated by the sterilizer 16 may pass through the light diffuser 15 to be irradiated into the storage space of the storage device 1. In order to minimize loss of ultraviolet light passing through the light diffuser 15, the light diffuser 15 may be made of a transparent or translucent material or a material having excellent transmittance of ultraviolet light.

The pet care device 5, the plurality of care tools 6, the cleaning module 8, and the base 7 may be sterilized via light emitted from the sterilizer 16 and passing through the light diffuser 15 during storage. In addition, the ultraviolet light passing through the light diffuser 15 may be irradiated to the first body 11 and the second body 21.

The first body 11 and the second body 21 are made of or coated with a metallic material or a material having excellent reflectance such that the ultraviolet light passing through the light diffuser 15 is reflected in the storage space. Alternatively, the first body 11 and the second body 21 are made of a material having excellent diffusivity such that ultraviolet light passing through the light diffuser 15 is further diffused. Materials having excellent reflectance or diffusivity of ultraviolet light may be coated on the upper or inner surface of the first body 11 and the lower or inner surface of the second body 21. Alternatively, the first body 11 and the second body 21 are formed of materials having excellent reflectance or diffusivity of ultraviolet light. In addition, the inner surface of the first case 10 and the second case 20 may be coated with materials excellent in reflectance or diffusion of ultraviolet light.

The ultraviolet light A passing through the light diffuser 15 may be reflected or diffused by the first body 11 and the second body 21 (as shown by ultraviolet light B) so that the pet care device 5, the plurality of care tools 6, the cleaning module 8, and the base 7 stored in the storage space may be sterilized uniformly. In addition, outer surfaces of the pet care device 5, the plurality of care tools 6, cleaning module 8, and cradle 7 may be coated with materials excellent in reflectance or diffusivity of ultraviolet light so that the ultraviolet light may be further diffused and reflected.

A part of the pet care device 5 in the care device seating groove 221 may be provided to face the light diffuser 15. When the ultraviolet sterilizer 5b of the pet care device 5 is provided to face the light diffuser 15, the sterilizer 5b may be turned on, and ultraviolet light from the sterilizer 5b may be passed through the light diffuser 15. When the care tools 6 are mounted on the pet care device 5, the care tools 6 may be efficiently sterilized by ultraviolet light from both the sterilizer 16 in the storage device 1 and the sterilizer 5b in the pet care device 5 passing through the light diffuser 15.

The ultraviolet light generated by the sterilizer 16 may have a wavelength that may be harmful to a user's health or eyes if irradiated toward the user. Therefore, the sterilizer 16 may be controlled to operate depending on whether the storage device 1 is open. There may be a sensor in the locking device 40 that senses whether the locking protrusion 411 is engaged with the protrusion groove 421 (FIG. 2).

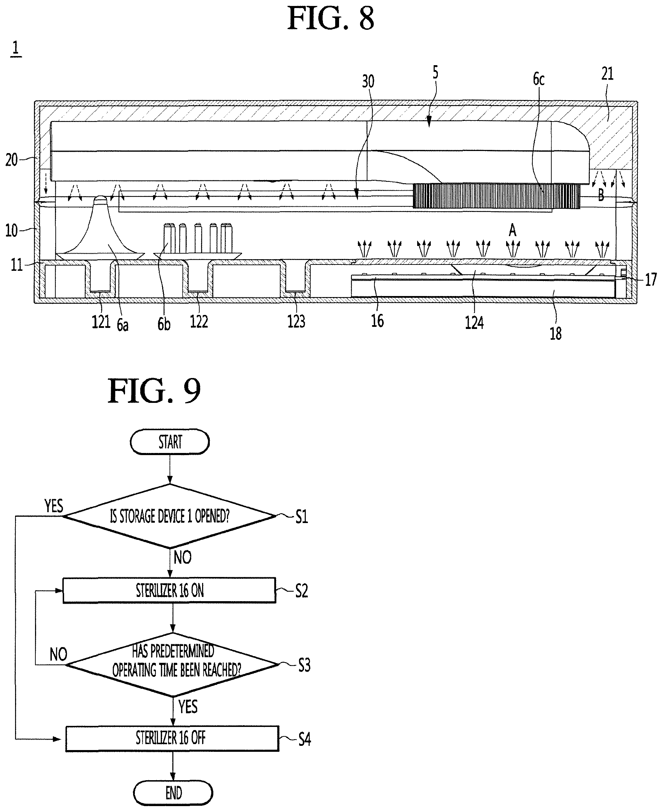

Referring to FIG. 9, a method for controlling the sterilizer 16 may start by first determining whether the storage device 1 is open (S1). The first case 10 and/or the second case 20 of the storage device 1 may include a sensor to detect whether the storage device 1 is open. A controller in the circuit board 17 (or alternatively, a controller on another circuit board in the storage device 1 electrically coupled to the circuit board 17) may determine whether the storage device 1 is opened based on a detection of the sensor.

When it is determined that the storage device 1 is open, the power of the sterilizer 16 may be turned off to prevent generation of ultraviolet light from the sterilizer 16 (S4). When it is determined that the storage device 1 is closed, the sterilizer 16 may be turned on to generate ultraviolet light (S2). The pet care device 5, the plurality of care tools 6, the cleaning module 8, and the base 7 are stored in the storage device 1 and sterilized by the ultraviolet light of the sterilizer 16.

There may further be a sensor in the plurality of seating grooves 12 and 22 to sense whether the pet care device 5, the plurality of care tools 6, the cleaning module 8, and the base 7 are provided within their respective seating grooves 222, 121, 122, 123, 221, and 124 and 223. An operation of the sterilizer 16 may be adjusted based on whether the pet care device 5, plurality of care tools 6, cleaning module 8, and base 7 are provided within the plurality of seating grooves 12 and 22 in addition to whether the storage device 1 is open. As an example, when the storage device 1 is closed and when at least one of the pet care device 5, plurality of care tools 6, cleaning module 8, and base 7 are positioned in the proper respective seating groove 222, 121, 122, 123, 221, and 124 and 223, the sterilizer 16 may be operated to turn on.

For example, when the pet care device 5 is provided in the care device seating groove 221 and the storage device 1 is closed, the sterilizer 16 may be turned on to sterilize the pet care device 5. On the contrary, when the storage device 1 is empty and none of the pet care device 5, plurality of care tools 6, cleaning module 8, and base 7 are provided in the plurality of seating grooves 11 and 12, the sterilizer 16 may be turned off to prevent unnecessary power consumption.

There may only be a sensor in the pet care device seating groove 221, and an operation of the sterilizer 16 may be primarily based on whether the storage device 1 is closed and on whether the pet care device 5 is mounted in the pet care device seating groove 221, regardless of whether the plurality of care tools 6, cleaning module 8, and base 7 are stored in the storage device 1. When the pet care device 5 is not mounted in the care device seating groove 221 and the storage device 1 is closed, the sterilizer 16 may be turned off to prevent unnecessary operation.

After the sterilizer 16 is turned on, it may operate for a predetermined operating time. An elapsed time may be measured from a time point at which the ultraviolet light starts to be irradiated from the sterilizer 16, and it may be detected whether the predetermined operating time of the sterilizer 16 is reached (S3). If the elapsed time reaches the predetermined operating time, the sterilizer 16 may be turned off (S4). If the elapsed time has not reached the predetermined operating time, then the sterilizer 16 remains on (S2). The predetermined operating time may be a preset time to prevent ultraviolet curing or unnecessary photochemical reactions from occurring in the devices stored in the storage device 1.

Referring to Figures 5 and 10A-10B, the storage device 1 may charge the pet care device 5 wirelessly or by wire during storage. For convenience of description, a wireless process will be described.

The storage device 1 may have a storage device battery 18 to supply power to charge the pet care device 5, a circuit board 17 to charge the storage device battery 18, and a power transmitter 23. The pet care device 5 may be provided in the care device seating groove 221 formed in the second body 21 of the second case 20. A care device battery 5c and a power receiver 5d may be provided in the pet care device 5. The power transmitter 23 may supply power to the power receiver 5d, the power receiver 5d may charge the care device battery 5c, and the care device battery 5c may operate the pet care device 5.

The power transmitter 23 may be provided at a position corresponding to or aligning with the power receiver 5d when the pet care device 5 is provided in the care device seating groove 221. Referring to FIG. 10A, the power transmitter 23 may be provided in a side surface of the second body 21, and the power receiver 5d may be provided in a side or end of the pet care device 5 to face the power transmitter 23. Referring to FIG. 10B, the power transmitter 23 may be provided in the inner surface of the second body 21, and the power receiver 5d may be provided at a corresponding side of the pet care device 5.

When the pet care device 5 is provided in the care device seating groove 221, the power receiver 5d may receive power transmitted from the power transmitter 23. The power transmitter 23 and the power receiver 5d may wirelessly transmit power using a method capable of supplying power wirelessly, such as a magnetic induction method or a magnetic revolution method. Bidirectional power transfer may be possible, and both the power receiver 5d and power transmitter 23 may be transceivers configured to be capable of both transferring and receiving power.

The care device battery 5c may additionally or alternatively be charged by wire. For example, a connector or terminal may be provided in each of the pet care device 5 and the care device seating groove 221, and the care device battery 5c may be charged through a connector.

When a cable is connected to the charging terminal 19 of the storage device 1, the cable may supply external power to charge the storage device battery 18. The cable may also supply power to the circuit board 17 to control an operation of the storage device 1 and to the power transmitter 23 to charge the care device battery 5c through the power receiver 5d. When the cable is not connected to the charging terminal 19 of the storage device 1, the care device battery 5c may be charged by the power of the storage device battery 18.

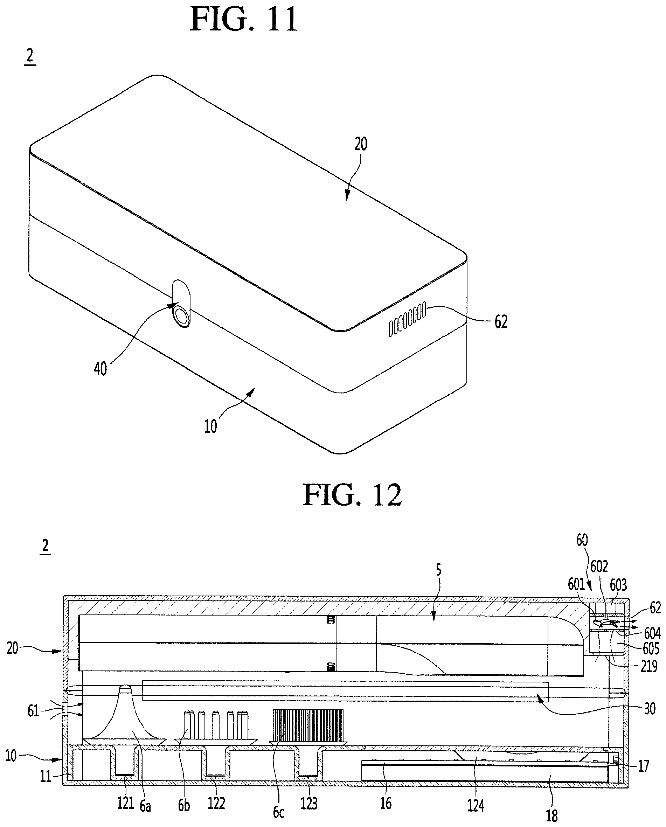

Referring to Figures 11 and 12, a storage device 2 according to a second embodiment may be substantially similar to the storage device 1 described with reference to Figures 1-10 except that the storage device 2 may include a cleaning module or assembly 60 to remove foreign matter present in an internal or storage space of the storage device 2. The cleaning assembly 60 may also be referred to as a filter assembly or suction cleaner. A detailed description of similarities between the storage device 2 and storage device 1 will be omitted.

The pet care device 5 may accumulate pet hair, fur, foreign matter, bacteria, etc. of the pet during management or treatment. Such pet hair, fur foreign matter, bacteria, etc. of the pet attached to the pet care device 5 may contaminate the internal space of the storage device 2 during storage. The cleaning assembly 60 may suck the foreign matter present in the internal space of the storage device 2 to keep the internal space clean. Although a method of sucking foreign matter is described, a method of cleaning the internal space using a brush, etc. is also possible.

The storage device 2 may include a plurality of inlets 61 through which external air flows into the internal space of the storage device 2 and a plurality of exhaust ports or outlets 62 through which air filtered by the cleaning assembly 60 is discharged. The inlet 61 may be provided at a first side or surface of the storage device 2, and the outlet 62 may be provided at a second side or surface of the storage device 2.

For example, the inlet 61 and the outlet 62 may be provided at opposite end surfaces of the storage device 2 and may have a predetermined width. When the inlet 61 and the outlet 62 are provided at opposite end surfaces of the storage device 2, a flow distance of air is increased, and a filtration or circulation of foreign matter present in the internal space of the storage device 2 may be increased. Positions of the inlet 61 and the outlet 62 are not limited to opposite end surfaces, however, may be changed to various positions on the storage device 2. For convenience of description, an example where the inlet 61 is formed on a surface of the first case 10 at a first end of the storage device 2 and where the outlet 62 is formed on a surface of the second case 20 at a second end of the storage device 2 will be described.

The cleaning assembly 60 may include a fan 601 to generate a flow of air, a rotation shaft 602 connected to the fan 601, and a motor 603 connected to the rotation shaft 602 to supply a rotational force. When the fan 601 is rotated, a suction force may be generated to pull or suck air through the inlet 61 and an output force may be generated to push or discharge the air to an outside of the storage device 2 through the outlet 62. The motor 603 may be operated by receiving power through the storage device battery 18 of the storage device 2.

The fan 601, the rotation shaft 602, and the motor 603 may be provided in any one of the first or second accommodation spaces 101 or 201 between the first case 10 and first body 11 or second case 20 and the second body 21. Alternatively or in addition thereto, the fan 601, the rotation shaft 602, and the motor 603 may be provided in any one of the first case 10 and the second case 20. For convenience of description, an example where the cleaning assembly 60 is provided in the second accommodation space 201 will be described.

The second accommodation space 201 in which the cleaning assembly 60 is provided may be configured to be partitioned from the first accommodation space 101, where a plurality of electronic components may be provided, to prevent foreign substances from contaminating the plurality of electronic components. Air introduced into the inlet 61 may be provided in the internal space of the storage device 2. An inlet 219 may be formed to align with an intake of the fan 601. Air introduced into the inlet 61 may pass through the internal space of the storage device 2 and then flow into the inlet 219, as shown by the arrows in FIG. 12.

The cleaning assembly 60 may include a filter 604 to filter foreign matter from the air introduced into the inlet 219 and a foreign matter reservoir 605 to store the foreign matter filtered by the filter 604. Based on a direction of flow of air, the foreign matter reservoir 605 may be provided downstream of the inlet 219, and the filter 604 may be provided downstream of the foreign matter reservoir 605 to filter foreign substances from the air passing through the foreign matter reservoir 605, and the foreign matter reservoir 605 may store the filtered foreign matter. The air passing through the filter 604 may be discharged to the outside through the outlet 62. The foreign matter reservoir 605 and the filter 604 may be configured to be detachable so that the foreign matter stored in the foreign matter reservoir 605 or attached to the filter 604 may be easily discharged or removed. The fan 601 may be provided downstream of the filter 604 and may be provided upstream of the outlet 62. When the fan 601 is rotated, air may be forced to flow from the inlet 61 toward the outlet 62.

It will be understood that when an element or layer is referred to as being "on" another element or layer, the element or layer can be directly on another element or layer or intervening elements or layers. In contrast, when an element is referred to as being "directly on" another element or layer, there are no intervening elements or layers present. As used herein, the term "and/or" includes any and all combinations of one or more of the associated listed items.

It will be understood that, although the terms first, second, third, etc., may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section could be termed a second element, component, region, layer or section without departing from the teachings of the present invention.

Spatially relative terms, such as "lower", "upper" and the like, may be used herein for ease of description to describe the relationship of one element or feature to another element(s) or feature(s) as illustrated in the figures. It will be understood that the spatially relative terms are intended to encompass different orientations of the device in use or operation, in addition to the orientation depicted in the figures. For example, if the device in the figures is turned over, elements described as "lower" relative to other elements or features would then be oriented "upper" relative to the other elements or features. Thus, the exemplary term "lower" can encompass both an orientation of above and below. The device may be otherwise oriented (rotated 90 degrees or at other orientations) and the spatially relative descriptors used herein interpreted accordingly.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Embodiments of the disclosure are described herein with reference to crosssection illustrations that are schematic illustrations of idealized embodiments (and intermediate structures) of the disclosure. As such, variations from the shapes of the illustrations as a result, for example, of manufacturing techniques and/or tolerances, are to be expected. Thus, embodiments of the disclosure should not be construed as limited to the particular shapes of regions illustrated herein but are to include deviations in shapes that result, for example, from manufacturing.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Any reference in this specification to "one embodiment," "an embodiment," "example embodiment," etc., means that a particular feature, structure, or characteristic described in connection with the embodiment is included in at least one embodiment of the invention. The appearances of such phrases in various places in the specification are not necessarily all referring to the same embodiment. Further, when a particular feature, structure, or characteristic is described in connection with any embodiment, it is submitted that it is within the purview of one skilled in the art to effect such feature, structure, or characteristic in connection with other ones of the embodiments.

## Claims

1. A storage device having an inner space for storing a pet care device (5), comprising:
a first case (10) having a first inner body (11) and a sterilizer (16), the sterilizer (16) being configured to emit ultraviolet light to the pet care device (5);
a second case (20) having a second inner body (21) being configured to accommodate the pet care device (5); and
a hinge (30) rotatably connecting first sides of the first and second cases (10, 20), wherein the inner space is formed inside the first case (10) and the second case (20);
**characterized in that** the first and the second inner bodies (11, 21) include a material capable of diffusing ultraviolet light.

2. The storage device of claim 1, wherein the first and second cases (10, 20) have second sides opposite the first sides such that the storage device is closed when the second sides contact each other and the storage device is opened when the second sides are separated from each other, and when the storage device is closed the sterilizer (16) emits ultraviolet light, and when the storage device is opened, the sterilizer (16) does not emit ultraviolet light.

3. The storage device of claim 1, wherein the second body (21) includes a pet care device seating groove (221) being configured to accommodate the pet care device (5).

4. The storage device of claim 3, wherein the first body (11) includes a plurality of care tool seating grooves (121, 122, 123) being configured to accommodate care tools (6, 6a to 6c).

5. The storage device of claim 3, wherein the sterilizer (16) is arranged in the first case (10) to face the pet care device seating groove (221) in the second body (21).

6. The storage device of claim 4, wherein the first case (10) includes a space (101) formed between an inner surface of the first case (10) and an inner surface of the first body (11), an opening formed in the inner surface of the first body (11), and a light diffuser (15) provided in the opening, and the sterilizer (16) is provided in the space to emit light through the light diffuser (15).

7. The storage device of claim 6, wherein the space is configured to include a battery (18) to supply power to the sterilizer (16) and a circuit board (17) to control an operation of the sterilizer (16).

8. The storage device of claim 7, wherein the first case (10) includes a charging terminal (19) to charge the battery (18).

9. The storage device of any one of claims 1 to 8, wherein the first case (10) includes a rib (41) and a locking protrusion (411) and the second case (20) includes an insertion groove (42) in which the rib (41) is inserted and a protrusion groove (421) in which the locking protrusion (411) is inserted.

10. The storage device of any one of claims 1 to 9, further comprising a cleaning assembly (60) to suction and filter foreign matter present in an inner space between the first and second bodies (11, 21).

11. The storage device of claim 10, wherein the cleaning assembly (60) includes:
an inlet (61) to allow air to flow to the internal space from an external space outside the storage device (2);
an outlet (62) to allow air to flow from the internal space to the external space; and
a fan (601) to suction air through the inlet (61) and discharge air toward the outlet (62).

12. The storage device of claim 11, wherein the cleaning assembly (60) further includes a filter (604) to filter foreign matter from air discharged through the outlet (62).

13. The storage device of claim 12, wherein the cleaning assembly (60) further includes a foreign matter reservoir (605) to store the foreign matter filtered by the filter (604).

14. The storage device of claim 11, 12 or 13, wherein the first and second cases (10, 20) have first ends and second ends opposite the first ends, wherein the inlet (61) is provided at the first end of the first case (10), and the outlet (62) is provided at the second end of the second case (20).

## Patentansprüche

1. Aufbewahrungsvorrichtung mit einem Innenraum zum Aufbewahren einer Haustier-Pflegevorrichtung (5), die aufweist:
ein erstes Gehäuse (10) mit einem ersten Innenkörper (11) und einem Sterilisator (16), wobei der Sterilisator (16) so konfiguriert ist, dass er Ultraviolettlicht zur Haustier-Pflegevorrichtung (5) abstrahlt;
ein zweites Gehäuse (20) mit einem zweiten Innenkörper (21), der so konfiguriert ist, dass er die Haustier-Pflegevorrichtung (5) aufnimmt; und
ein Scharnier (30), das erste Seiten des ersten und zweiten Gehäuses (10, 20) drehbar verbindet, wobei der Innenraum innerhalb des ersten Gehäuses (10) und des zweiten Gehäuses (20) gebildet ist;
**dadurch gekennzeichnet, dass** der erste und der zweite Innenkörper (11, 21) ein Material aufweisen, das Ultraviolettlicht streuen kann.

2. Aufbewahrungsvorrichtung nach Anspruch 1, wobei das erste und zweite Gehäuse (10, 20) zweite Seiten entgegengesetzt zu den ersten Seiten haben, so dass die Aufbewahrungsvorrichtung geschlossen ist, wenn die zweiten Seiten einander berühren, und die Aufbewahrungsvorrichtung geöffnet ist, wenn die zweiten Seiten voneinander getrennt sind, und bei geschlossener Aufbewahrungsvorrichtung der Sterilisator (16) Ultraviolettlicht abstrahlt und bei geöffneter Aufbewahrungsvorrichtung der Sterilisator (16) kein Ultraviolettlicht abstrahlt.

3. Aufbewahrungsvorrichtung nach Anspruch 1, wobei der zweite Körper (21) eine Aufnahmevertiefung (221) für eine Haustier-Pflegevorrichtung aufweist, die so konfiguriert ist, dass sie die Haustier-Pflegevorrichtung (5) aufnimmt.

4. Aufbewahrungsvorrichtung nach Anspruch 3, wobei der erste Körper (11) mehrere Aufnahmevertiefungen (121, 122, 123) für Pflegewerkzeuge aufweist, die so konfiguriert sind, dass sie Pflegewerkzeuge (6, 6a bis 6c) aufnehmen.

5. Aufbewahrungsvorrichtung nach Anspruch 3, wobei der Sterilisator (16) im ersten Gehäuse (10) so angeordnet ist, dass er zur Aufnahmevertiefung (221) für die Haustier-Pflegevorrichtung im zweiten Körper (21) weist.

6. Aufbewahrungsvorrichtung nach Anspruch 4, wobei das erste Gehäuse (10) aufweist: einen Raum (101), der zwischen einer Innenfläche des ersten Gehäuses (10) und einer Innenfläche des ersten Körpers (11) gebildet ist, eine Öffnung, die in der Innenfläche des ersten Körpers (11) gebildet ist, und einen Lichtdiffusor (15), der in der Öffnung vorgesehen ist, und der Sterilisator (16) im Raum vorgesehen ist, um Licht durch den Lichtdiffusor (15) abzustrahlen.

7. Aufbewahrungsvorrichtung nach Anspruch 6, wobei der Raum so konfiguriert ist, dass er eine Batterie (18) zur Stromversorgung des Sterilisators (16) und eine Leiterplatte (17) zur Steuerung eines Betriebs des Sterilisators (16) aufweist.

8. Aufbewahrungsvorrichtung nach Anspruch 7, wobei das erste Gehäuse (10) einen Ladeanschluss (19) zum Laden der Batterie (18) aufweist.

9. Aufbewahrungsvorrichtung nach einem der Ansprüche 1 bis 8, wobei das erste Gehäuse (10) eine Rippe (41) und einen Verriegelungsvorsprung (411) aufweist und das zweite Gehäuse (20) eine Einführnut (42), in die die Rippe (41) eingeführt wird, und eine Vorsprungnut (421) aufweist, in die der Verriegelungsvorsprung (411) eingeführt wird.

10. Aufbewahrungsvorrichtung nach einem der Ansprüche 1 bis 9, die ferner eine Reinigungsanordnung (60) aufweist, um Fremdstoffe anzusaugen und zu filtern, die in einem Innenraum zwischen dem ersten und zweiten Körper (11, 21) vorhanden sind.

11. Aufbewahrungsvorrichtung nach Anspruch 10, wobei die Reinigungsanordnung (60) aufweist:
einen Einlass (61), damit Luft aus einem Außenraum außerhalb der Aufbewahrungsvorrichtung (2) zum Innenraum strömen kann;
einen Auslass (62), damit Luft aus dem Innenraum zum Außenraum strömen kann; und
einen Lüfter (601), um Luft durch den Einlass (61) anzusaugen und Luft zum Auslass (62) abzugeben.

12. Aufbewahrungsvorrichtung nach Anspruch 11, wobei die Reinigungsanordnung (60) ferner einen Filter (604) aufweist, um Fremdstoffe aus Luft zu filtern, die durch den Auslass (62) abgegeben wird.

13. Aufbewahrungsvorrichtung nach Anspruch 12, wobei die Reinigungsanordnung (60) ferner ein Fremdstofflager (605) aufweist, um die durch den Filter (604) gefilterten Fremdstoffe zu lagern.

14. Aufbewahrungsvorrichtung nach Anspruch 11, 12 oder 13, wobei das erste und zweite Gehäuse (10, 20) erste Enden und zweite Enden entgegengesetzt zu den ersten Enden haben, wobei der Einlass (61) am ersten Ende des ersten Gehäuses (10) vorgesehen ist und der Auslass (62) am zweiten Ende des zweiten Gehäuses (20) vorgesehen ist.

## Revendications

1. Dispositif de stockage ayant un espace interne pour stocker un dispositif de soins pour animaux de compagnie (5), comprenant:
un premier boîtier (10) ayant un premier corps interne (11) et un stérilisateur (16), le stérilisateur (16) étant configuré pour émettre une lumière ultraviolette vers le dispositif de soins pour animaux de compagnie (5);
un second boîtier (20) ayant un second corps interne (21) qui est configuré pour recevoir le dispositif de soins pour animaux de compagnie (5); et
une charnière (30) reliant de manière rotative des premiers côtés des premier et second boîtiers (10, 20), dans lequel l'espace interne est formé à l'intérieur du premier boîtier (10) et du second boîtier (20);
**caractérisé en ce que** les premier et second corps internes (11, 21) incluent un matériau capable de diffuser la lumière ultraviolette.

2. Dispositif de stockage selon la revendication 1, dans lequel les premier et second boîtiers (10, 20) ont des seconds côtés opposés aux premiers côtés de sorte que le dispositif de stockage est fermé lorsque les seconds côtés sont en contact l'un avec l'autre et le dispositif de stockage est ouvert lorsque les seconds côtés sont séparés l'un de l'autre, et lorsque le dispositif de stockage est fermé, le stérilisateur (16) émet de la lumière ultraviolette, et lorsque le dispositif de stockage est ouvert, le stérilisateur (16) n'émet pas de lumière ultraviolette.

3. Dispositif de stockage selon la revendication 1, dans lequel le second corps (21) inclut une rainure de logement de dispositif de soins pour animaux de compagnie (221) qui est configurée pour recevoir le dispositif de soins pour animaux de compagnie (5).

4. Dispositif de stockage selon la revendication 3, dans lequel le premier corps (11) inclut une pluralité de rainures de logement d'outils de soins (121, 122, 123) qui sont configurées pour recevoir des outils de soins (6, 6a à 6c).

5. Dispositif de stockage selon la revendication 3, dans lequel le stérilisateur (16) est agencé dans le premier boîtier (10) pour faire face à la rainure de logement de dispositif de soins pour animaux de compagnie (221) dans le second corps (21).

6. Dispositif de stockage selon la revendication 4, dans lequel le premier boîtier (10) inclut un espace (101) formé entre une surface interne du premier boîtier (10) et une surface interne du premier corps (11), une ouverture formée dans la surface interne du premier corps (11), et un diffuseur de lumière (15) disposé dans l'ouverture, et le stérilisateur (16) est disposé dans l'espace pour émettre de la lumière à travers le diffuseur de lumière (15).

7. Dispositif de stockage selon la revendication 6, dans lequel l'espace est configuré pour inclure une batterie (18) pour fournir de l'énergie au stérilisateur (16) et une carte de circuit (17) pour commander un fonctionnement du stérilisateur (16).

8. Dispositif de stockage selon la revendication 7, dans lequel le premier boîtier (10) inclut une borne de charge (19) pour charger la batterie (18).

9. Dispositif de stockage selon l'une quelconque des revendications 1 à 8, dans lequel le premier boîtier (10) inclut une nervure (41) et une protubérance de verrouillage (411) et le second boîtier (20) inclut une rainure d'insertion (42) dans laquelle la nervure (41) est insérée et une rainure à protubérance (421) dans laquelle la protubérance de verrouillage (411) est insérée.

10. Dispositif de stockage selon l'une quelconque des revendications 1 à 9, comprenant en outre un ensemble de nettoyage (60) pour aspirer et filtrer les matières étrangères présentes dans un espace interne entre les premier et second corps (11, 21).

11. Dispositif de stockage selon la revendication 10, dans lequel l'ensemble de nettoyage (60) inclut:
une entrée (61) pour permettre à l'air de s'écouler vers l'espace interne depuis un espace externe à l'extérieur du dispositif de stockage (2);
une sortie (62) pour permettre à l'air de s'écouler de l'espace interne vers l'espace externe; et
un ventilateur (601) pour aspirer l'air à travers l'entrée (61) et évacuer l'air vers la sortie (62).

12. Dispositif de stockage selon la revendication 11, dans lequel l'ensemble de nettoyage (60) inclut en outre un filtre (604) pour filtrer les matières étrangères de l'air évacué par la sortie (62).

13. Dispositif de stockage selon la revendication 12, dans lequel l'ensemble de nettoyage (60) inclut en outre un réservoir à matières étrangères (605) pour stocker les matières étrangères filtrées par le filtre (604).

14. Dispositif de stockage selon la revendication 11, 12 ou 13, dans lequel les premier et second boîtiers (10, 20) ont des premières extrémités et des secondes extrémités opposées aux premières extrémités, dans lequel l'entrée (61) est disposée à la première extrémité du premier boîtier (10), et la sortie (62) est disposée à la seconde extrémité du second boîtier (20).
